(19) Europäisches Patentamt
European Patent Office
Office européen des brevets

(11) Veröffentlichungsnummer : **0 143 385**
**B1**

(12) # EUROPÄISCHE PATENTSCHRIFT

(45) Veröffentlichungstag der Patentschrift : **15.03.89**

(51) Int. Cl.[4] : **C 07 H 13/06**, C 07 H 13/12, A 61 K 31/70

(21) Anmeldenummer : **84113619.5**

(22) Anmeldetag : **12.11.84**

(54) **Phosphorylierte Glycosylamide, -harnstoffe, -carbamate und -thiocarbamate, Verfahren zu ihrer Herstellung sowie ihre Verwendung.**

(30) Priorität : **23.11.83 DE 3342308**
**02.02.84 DE 3403495**

(43) Veröffentlichungstag der Anmeldung : **05.06.85 Patentblatt 85/23**

(45) Bekanntmachung des Hinweises auf die Patenterteilung : **15.03.89 Patentblatt 89/11**

(84) Benannte Vertragsstaaten : **AT BE CH DE FR GB IT LI NL SE**

(56) Entgegenhaltungen :
**EP–A– 0 091 645**
**CHEMICAL ABSTRACTS, Band 87, Nr. 11, 12. September 1977, Seite 621, Zusammenfassung Nr. 85185h, Columbus, Ohio, US; G. CHETTUR et al.: "A new chemical synthesis of 2-amino-(N-d-ribofuranosyl)acetamide 5-phosphate", & CARBOHYDR. RES. 1977, 56(1), 75-86**

(73) Patentinhaber : **BAYER AG**
**Konzernverwaltung RP Patentabteilung**
**D-5090 Leverkusen 1 Bayerwerk (DE)**

(72) Erfinder : **Lockhoff, Oswald, Dr.**
**Morgengraben 14**
**D-5000 Koeln 80 (DE)**
Erfinder : **Krüger, Bernd-Wieland, Dr.**
**Sillerstrasse 49**
**D-5600 Wuppertal 1 (DE)**
Erfinder : **Stadler, Peter, Dr.**
**Am Ideck 8**
**D-5657 Haan 1 (DE)**
Erfinder : **Paessens, Arnold, Dr.**
**Stresemannstrasse 56**
**D-5657 Haan 1 (DE)**
Erfinder : **Streissle, Gert, Dr.**
**Am Hochsitz 17**
**D-5600 Wuppertal 1 (DE)**
Erfinder : **Taylor, Peter, Dr.**
**Molecular Diagnostics Inc. 400 Morgan Lane**
**West Haven Connecticut 06516 (US)**
Erfinder : **Zeiler, Hans-Joachim, Dr.**
**Elsbeeker Strasse 46**
**D-5620 Velbert 15 (DE)**
Erfinder : **Metzger, Karl-Georg, Dr.**
**Pahlkestrasse 75**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 143 385 B1

Jouve, 18, rue St-Denis, 75001 Paris, France

**Beschreibung**

Die Erfindung betrifft neue monophosphorylierte N-Glycosylamide, N-Glycosylharnstoffe, N-Glycosylcarbamate und N-Glycosylthiocarbamate, Verfahren zu ihrer Herstellung sowie ihre Verwendung als Arzneimittel.

In der EP-A-91645 werden strukturell ähnliche, nicht phosphorylierte Verbindungen, deren Herstellung und Anwendung beschrieben.

Die neuen Verbindungen entsprechen der allgemeinen Formel I

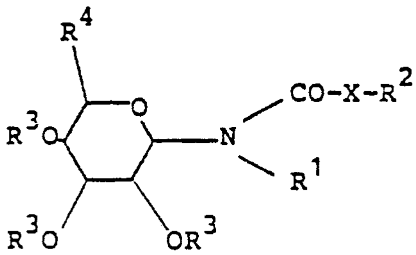

(I)

Hierin bedeutet $R^1$ einen gegebenenfalls substituierten geradkettigen oder verzweigten gesättigten oder einfach oder mehrfach ungesättigten Alkylrest, einen gegebenenfalls substituierten, gesättigten oder ein- oder mehrfach ungesättigten Cycloalkylrest, einen gegebenenfalls substituierten Arylrest oder Aralkylrest,

X bedeutet $CH_2$, O, S, NH oder N-Alkyl, wobei dieser Alkylrest bis zu 20, vorzugsweise 1-10 Kohlenstoffatome enthalten kann,

$R^2$ hat die Bedeutung von $R^1$ und kann zusätzlich für Wasserstoff stehen, wenn X für $CH_2$ steht,

$R^3$ bedeutet unabhängig voneinander Wasserstoff, einen Acylrest mit 1 bis 20, vorzugsweise 1-10 Kohlenstoffatomen, einen Alkylrest mit 1 bis 20, vorzugsweise 1-10 Kohlenstoffatomen, einen Silylrest und/oder einen Phosphorsäure- oder Phosphorsäureesterrest und

$R^4$ bedeutet Wasserstoff, Methyl oder $—CH_2—OR^3$ mit der Maßgabe, daß immer einer der Reste $R^3$ einen Phosphorsäure- oder Phosphorsäureesterrest darstellt.

Der Rest $R^1$ in der Bedeutung Alkyl hat bis zu 40, vorzugsweise bis zu 20 C-Atome und ganz besonders bevorzugt 10-20 C-Atome.

Beispiele für gesättigte Alkylreste $R^1$ sind Methyl, Ethyl, n-Propyl, i-Propyl, n-Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Eicosyl, Docosyl, Tetracosyl, Triacontyl, Ethylpentyl, Methyldecyl, i-Propyldecyl, Methyltridecyl, Pentylhexadecyl, 1-Dodecylhexadecyl, 2-Dodecylhexadecyl, 3-Dodecylhexadecyl, 1-Hexadecyloctadecyl, 2-Hexadecyloctadecyl, 3-Hexadecyloctadecyl, 4-Hexadecyloctadecyl, 1-Octadecyleicosyl, 2-Octadecyleicosyl.

Ungesättigte Alkylreste $R^1$ sind beispielsweise Ethenyl, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 8,11-Heptadecandienyl, 8,11,14-Heptadecantrienyl.

Im allgemeinen sind unter den ungesättigten Resten die längerkettigen bevorzugt, speziell die einfach oder zweifach ungesättigten Alkenyle mit 10-20 Kohlenstoffatomen.

Die ungesättigten Kohlenwasserstoffreste können dabei als reine cis- oder trans-Isomere oder auch als Isomerengemische vorliegen.

$R^1$ in der Bedeutung Cycloalkyl steht bevorzugt für Reste mit 3-7 C-Atomen.

Beispiele für Cycloalkylreste sind Cyclopropyl, Cyclobutyl, Cyclopentyl, Cyclohexyl, Cycloheptyl, Cyclohexylcyclohexyl, Cyclopentylcycloheptyl.

Beispiele für ungesättigte cyclische Reste $R^1$ sind Cyclopentenyl, Cyclohexenyl und Cycloheptenyl. Bevorzugte Substituenten für Cycloalkylreste sind Alkylreste mit bis zu 8 C-Atomen.

Beispielhaft hierfür seien genannt :

Methylcyclopentyl, Ethylcyclopentyl, n-Propylcyclopentyl, i-Propylcyclopentyl, Butylcyclopentyl, Octylcyclopentyl, Methylcyclohexyl, Ethylcyclohexyl, Propylcyclohexyl, Butylcyclohexyl, Hexylcyclohexyl, Decylcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexyl, Cyclohexyldecyl, Cyclopentylcyclohexylethyl, Cyclohexylcyclopentylethyl und Cyclohexylcyclohexylethyl.

Die Substituenten können jeweils cis- oder transständig angeordnet sein.

$R^1$ in der Bedeutung Aralkyl kann verschiedenartig sein. So kann zum einen der Arylrest, der vorzugsweise aus 6 oder 10 Kohlenstoffatomen besteht, als Substituent der Alkylkette vorliegen, er kann jedoch auch in die Alkylkette eingefügt sein. Auf diese Weisen gebunden, können vorzugsweise 1-3 Arylreste vorliegen. Die Arylreste können zusätzlich substituiert sein und zwar vorzugsweise durch 1 oder

2 Gruppen an der Reihe Nitro, Niederalkyl ($C_1$-$C_4$) oder $C_1$-$C_4$-Alkoxy oder können bis zu 5 Halogenatome, vorzugsweise Fluor und Chlor tragen.

$R^1$ in der Bedeutung Aryl steht bevorzugt für $C_6$-, $C_{10}$- oder $C_{12}$-aromatische Reste. Diese können gegebenenfalls substituiert sein, vorzugsweise durch $C_1$-$C_4$-Alkyl, $C_1$-$C_4$-Alkoxy oder Nitro, jeweils vorzugsweise 1-2 Substituenten, oder durch 1-5 Halogenatome, vorzugsweise Fluor oder Chlor.

Beispiele für Aryl- und Aralkylreste $R^1$ sind Phenyl, Diphenyl, p-Nitrophenyl, Ethylphenyl, para-Methoxyphenyl, 2,4-Dichlorphenyl, Benzyl, para-Methoxybenzyl, Phenylethyl, Phenylhexyl, Tolylheptyl, z-Phenyltetradecyl und 14-Phenyltetradecyl.

In den Resten $R^1$, insbesondere in der Bedeutung Alkyl und Alkenyl können auch einzelne Methylengruppen oder Methingruppen durch Sauerstoff-, Schwefel- oder Stickstoffatome ersetzt sein. Bei einer Unterbrechung der Alkylkette durch N kann dieses Stickstoffatom entweder H oder einen Alkylrest mit bis zu 20 vorzugsweise bis zu 6 Kohlenstoffatomen oder einen CO-Alkylrest mit bis zu 20 vorzugsweise bis zu 6 C-Atomen tragen.

Im allgemeinen können bis zu 5, vorzugsweise ein bis drei Methylen- oder Methingruppen derart ersetzt sein.

Die Reste $R^1$ können substituiert sein, vorzugsweise durch $C_6$-, $C_{10}$- oder $C_{12}$-Aryl, Halogen, vorzugsweise F, Cl oder Br, Amino, $C_1$-$C_6$-Alkylamino, Di-$C_1$-$C_6$-alkylamino, OH, $C_1$-$C_6$-Alkoxy, O—CO—R' oder NH-COR' wobei R' jeweils $C_1$-$C_6$-Alkyl darstellt. Wenn $R^1$ substituiert ist, so liegen vorzugsweise 1 oder 2 gleiche oder verschiedene Substituenten vor.

Beispiele, in denen die Reste $R^1$ in Formel I durch Sauerstoff-, Schwefel- und/oder Stickstoffatome unterbrochene Kohlenwasserstoffreste darstellen oder die genannten, darin enthaltende Atome substituiert sind, sind Methoxyethyl, Methoxyethoxyethyl, Dimethylaminoethyloxyethyl, Dibutylaminohexyl, Mercaptoethyl, Oxobutyl, Aminodecyl, N-Methylaminodecyl, Chlorethyl, Fluormethyl, 2-Hydroxy-Tetradecyl, 7-Phenyloctadecyl, 16-Phenylhexadecyl, 2-Brompropyl, 8-Bromdodecyl, 16-Chlorhexadecyl, 2-Aminotetradecyl, 16-Aminohexadecyl, 4-(Butylamino)-hexadecyl, 8-(Dimethylamino)-octadecyl, 8-Hydroxyoctadecyl, 4-Methoxydodecyl, 5-(Butylcarbonyloxy) tridecyl, 2-(Tridecylcarbonyloxy) tetradecyl, 2-(Tridecylcarbonylamido)-tetradecyl, 2-Hydroxyoctadec-8-en-1-yl, 2-Aminooctadec-8-en-1-yl, Tridecylcarbonyloxy-octadec-8-en-1-yl.

In dem Rest $R^3$ können der Acylrest und der Alkylrest ihrerseits substituiert sein. Hier kommen besonders 1-2 Substituenten aus der Gruppe Halogen (F, Cl, Br, vorzugsweise Cl) ; Aryl, vorzugsweise Phenyl oder durch 1-2 Halogen oder $C_1$-$C_4$-Alkoxy substituiertes Phenyl ; oder $C_1$-$C_4$-Alkoxy in Betracht.

Beispiele für $R^3$ aus der Acylreihe sind Acetyl, Propionyl, Butyryl, Pivaloyl, Benzoyl, p-Methoxybenzoyl, 2,4-Dichlorbenzoyl, Caproyl, Myristoyl, Stearoyl.

Beispiele für $R^3$ aus der Alkylreihe Methyl, Ethyl, Propyl, i-Propyl, Heptyl, Decyl, Octadecyl, Allyl, 1-Propenyl, Benzyl, p-Methoxybenzyl, Ethyliden, Isopropyliden, Isobutyliden, Benzyliden, p-Methoxybenzyliden, 1-Methoxyethyliden.

Beispiele für $R^3$ aus der Silylethergruppe sind Trimethylsilyl, Diethylpropylsilyl.

In der Bedeutung von $R^3$ ist unter Phosphorsäurerest die Gruppe $-PO(OH)_2$ zu verstehen, unter Phosphorsäureesterrest die Gruppen

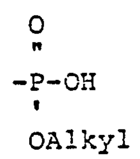
oder
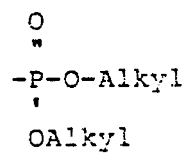

worin « Alkyl » jeweils unabhängig einen geradkettigen oder verzweigten Alkylrest mit 1-20, vorzugsweise mit 1-5 C-Atomen steht.

Wie in Formel I ersichtlich ist, liegt den erfindungsgemäßen Verbindungen ein Zuckermolekül zugrunde, bevorzugt Pentosen und Hexosen.

Beispiele für solche Zucker sind Ribose, Arabinose, Xylose, Lyxose, Allose, Altrose, Glucose, Mannose, Gulose, Idose, Galactose oder Talose, sowohl in der D-Reihe als auch in der L-Reihe.

Die Verbindungen gemäß Formel I enthalten als einen der Reste $R^3$ oder im Rest $R^4$ immer eine Phosphorsäuregruppe oder eine Phosphorestergruppe, die esterartig mit einer Hydroxylgruppe des Zuckerteils verbunden sind.

In dem Fall, in dem Pentosen die Zuckerbestandteile der Verbindungen gemäß Formel I sind, ist also eine der sekundären zum Zuckerring gehörigen Hydroxylgruppen phosphoryliert. In dem Fall, in dem Hexosen die Zuckerbestandteile von Verbindungen gemäß Formel I bilden, ist entweder eine der zum Cyclus gehörigen sekundären Hydroxylgruppen oder aber die Hydroxylgruppe der exocyclischen Hydroxymethylgruppe phosphoryliert.

Die nicht phosphorylierten Hydroxylgruppen liegen entweder frei, d. h. unblockiert, oder blockiert durch Ester-, Ether- und/oder Silylreste vor.

Die Verbindungen der Formel I enthalten mehrere chirale C-Atome und liegen als optisch reine Diastereomere oder als Diastereomerengemische vor.

Die Erfindung betrifft auch Verfahren zur Herstellung der Verbindungen gemäß Formel I. Hierbei setzt

man die von Formel I umfaßten Zucker entweder in freier, d. h. ungeschützter Form, oder in Form geschützter und gegebenenfalls partiell phosphorylierter und/oder gegebenenfalls aktivierter Derivate zunächst um mit einer Aminoverbindung $R^1$-$NH_2$, entweder in freier Form oder in Form eines geeigneten Säureadditionssalzes, mit der vorstehenden Bedeutung von $R^1$. Die auf diese Weise erhaltenen Glycosylamine der Formel II

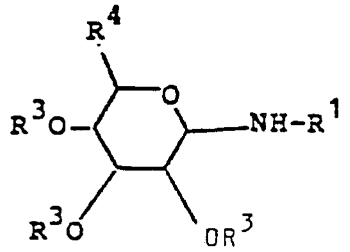

(II)

mit den vorstehend beschriebenen Bedeutungen von $R^1$, $R^3$ und $R^4$ werden dann mit einem — wie bei Acylierungsreaktionen üblich — aktivierten, an funktionellen Gruppen gegebenenfalls geschützten Carbonsäure- oder Kohlensäurederivat umgesetzt zum Glycosylamid, Glycosylharnstoff, Glycosylcarbamat oder Glycosylthiocarbamat. Falls nicht von vorneherein partiell phosphorylierte Zucker eingesetzt worden sind, wird im dritten Reaktionsschritt, der naturgemäß aus einer Reihe von Reaktionen bestehen kann, die Phosphatgruppe eingeführt. In den so erhaltenen Reaktionsprodukten werden gegebenenfalls vorhandene Schutzgruppen abgespalten und man erhält auf diese Weise die erfindungsgemäßen Verbindungen der Formel I, die, falls erforderlich, durch Chromatographie, Umkristallisieren, Extraktion o.ä. gereinigt werden können.

In einer bevorzugten Ausführungsform des erfindungsgemäßen Verfahrens wird in an sich bekannter Weise in einem ersten Verfahrensschritt ein unblockierter Zucker aus der Aldopentose- oder Aldohexosereihe mit einem Amin $R^1$-$NH_2$ zur Reaktion gebracht, wobei unter Wasserabspaltung ein Glycosylamin der Formel II entsteht. In diesem Fall steht für $R^3$ Wasserstoff und für $R^4$ entweder Wasserstoff, Methyl oder Hydroxymethyl.

Amine $R^1$-$NH^2$, die bei Raumtemperatur flüssig sind, können mit dem Zucker direkt, d.h. ohne Lösungsmittel, umgesetzt werden. Hierbei arbeitet man bei einer Temperatur zwischen 0 °C und 100 °C, vorzugsweise 25 °C und 70 °C. Als Katalysatoren sind Mineralsäuren wie z. B. Salzsäure oder Schwefelsäure oder kurzkettige Carbonsäuren wie Essigsäure oder Propionsäure geeignet, die man in Mengen von 0,001 bis 0,05 Äquivalenten einsetzt.

In jedem Fall ist es möglich, und bei Aminen $R^1$-$NH_2$, die bei Raumtemperatur fest sind auch zu bevorzugen, die Herstellung der Glycosylamine in Gegenwart eines Lösungsmittels durchzuführen. Man arbeitet dann vorzugsweise in Gegenwart eines unter den Reaktionsbedingungen inerten Verdünnungsmittels, welches vorzugsweise so beschaffen ist, daß sich zumindest entweder die Reaktionspartner oder das Reaktionsgemisch in ihm lösen.

Infrage kommen Alkohole wie Methanol, Ethanol, 1-Propanol, 2-Propanol, Ether wie Tetrahydrofuran und Dioxan, sowie auch Dimethylformamid, wobei der Zusatz von Wasser gegebenenfalls bevorzugt wird.

Die Reaktionstemperaturen bei Verwendung von Lösungsmitteln bei der Herstellung der Glycosylamine liegen zwischen —10 °C und 120 °C, vorzugsweise zwischen 30 °C und 70 °C.

Das betreffende Verdünnungsmittel kann wahlweise vor oder während der Reaktion zugegeben werden. Bei langkettigen Aminen $R^1$-$NH_2$ ist eine Zugabe vor der Reaktion zu bevorzugen.

Die wie vorstehend beschrieben hergestellten Glycosylamine kristallisieren entweder direkt oder nach dem Abkühlen aus und können durch Zusatz geeigneter, vorzugsweise weniger polarer Hilfsmittel wie Aceton, Diethylether, Cyclohexan, Essigester oder Petrolether, gegebenenfalls unter Kühlung, ausgefällt oder zur Kristallisation gebracht werden. Gegebenenfalls vorhandenes überschüssiges Amin $R^1$-$NH_2$ kann durch Waschen oder Umkristalisieren des Produktes auf an sich bekannte Weise entfernt werden. Die auf diese Weise erhaltenen Glycosylamine der Formel II werden im zweiten Reaktionsschritt selektiv am Stickstoffatom mit Carbonsäurederivaten oder Kohlensäurederivaten acyliert zu Glycosylamiden, Glycosylcarbamaten, Glycosylthiocarbamaten oder Glycosylharnstoffen.

Für den Fall, daß X in Formel I eine Methylengruppe darstellt, setzt man die entsprechenden Glycosylamine der Formel II um, mit 1 bis 10 Äquivalenten eines Carbonsäurederivats der Formel $R^2$—$CH_2$—CO—Y, in der $R^2$ die oben angegebene Bedeutung hat und Y Halogen oder eine bei Amidierungsreaktionen übliche Abgangsgruppe bedeutet, vorzugsweise einen aktivierenden Esterrest wie z. B. para-Nitrophenyl oder eine Gruppe O—CO—$R^2$ mit der obigen Bedeutung für $R^2$ oder eine Gruppe O—CO—O—$R^2$ mit der obigen Bedeutung von $R^2$. Dabei arbeitet man in organischen oder wäßrig-organischen Lösungsmitteln bei Temperaturen zwischen 0° und 50 °C, gegebenenfalls in Gegenwart einer Base.

Für den Fall, daß X in Formel I ein Sauerstoffatom darstellt, setzt man ein Glycosylamin der Formel II um mit 1 bis 5 Äquivalenten eines Halogen-kohlensäureesters Y—CO—O—$R^2$, wobei Y für Halogen, bevorzugt Chlor, steht und $R^2$ die oben beschriebene Bedeutung hat. Man arbeitet dabei in organischen

Lösungsmitteln bei Temperaturen zwischen —20 °C und 50 °C, gegebenenfalls in Gegenwart einer Base.

Für den Fall, daß X in Formel I ein Schwefelatom darstellt, läßt man ein Glycosylamin der Formel II reagieren mit einem Thiokohlensäurehalogenid-S-ester $R^2$—S—CO—Y, wobei Y für Halogen, bevorzugt Chlor, steht und $R^2$ die obige Bedeutung zukommt. Es wird in organischen Lösungsmitteln gearbeitet, gegebenenfalls in Gegenwart eines geeigneten Katalysators und/oder einer Base, die Reaktionstemperaturen liegen zwischen 0 °C und 70 °C.

Für den Fall, daß X in Formel I eine NH-Gruppe bedeutet, setzt man ein Glycosylamin der Formel II um mit 1 bis 5 Äquivalenten eines Isocyanats $R^2$-NCO mit der obengenannten Bedeutung von $R^2$. Es wird bevorzugt in organischen Lösungsmitteln umgesetzt, gegebenenfalls in Gegenwart eines Katalysators, die Reaktionstemperaturen liegen zwischen —20 °C und 50 °C.

Für den Fall, daß X in Formel I eine NH-Gruppe oder eine N-Alkylgruppe bedeutet, kann man auch ein Carbamat, das man durch Reaktion des Amins gemäß Formel II mit einem bevorzugt aromatischen Halogenameisensäureesters erhält, mit 1 bis 10 Äquivalenten eines primären Amins $R^2$—$NH_2$ oder eines sekundären Amins $R^2$-NH-Alkyl mit den vorstehend beschriebenen Bedeutungen für $R^2$ zur Reaktion bringen. Es wird bevorzugt bei 20 °C bis 80 °C in organischen Lösungsmitteln umgesetzt, gegebenenfalls in Gegenwart eines Katalysators.

Die Carbonsäure- bzw. Kohlensäurederivate werden vorzugsweise in Gegenwart eines Verdünnungsmittels mit den Glycosylaminen umgesetzt, in welchem die Reaktionspartner vollständig oder auch nur teilweise gelöst sind.

Es kommen organische oder anorganische Solventien infrage, vorzugsweise solche, die unter den Reaktionsbedingungen Nebenreaktionen möglichst herabsetzen oder verhindern. Man kann sowohl in organischen Lösungsmitteln wie Ethern, z. B. Tetrahydrofuran oder Dioxan, oder Alkoholen wie z. B. Ethanol oder Propanol, oder Ketonen wie Aceton oder Methylethylketon oder in Dimethylformamid oder Essigester oder Pyridin oder auch in Mischungen dieser Lösungsmittel untereinander und/oder mit Wasser arbeiten. Die Verwendung von wasserfreien Solventien ist im allgemeinen zu bevorzugen.

Die Umsetzungen mit den Carbonsäurederivaten oder den Kohlensäurederivaten können in Gegenwart basischer Hilfsstoffe durchgeführt werden. Verwendet werden können alle in der organischen Synthese üblichen basischen Verbindungen wie z. B. tertiäre aliphatische oder aromatische Amine wie Triethylamin oder Pyridin oder aber Alkali- oder Erdalkalihydroxide bzw. -carbonate wie Natriumhydroxid, Natriumcarbonat oder Calciumcarbonat. Die auf diese Weise erhaltenen N-Glycosylamide, -carbamate, -thiocarbamate oder -harnstoffe werden nach an sich bekannten Verfahren in Form von kristallinen oder amorphen Feststoffen oder als zähe Sirupe isoliert und, wenn notwendig, durch Kristallisation, Chromatographie, Extraktion usw. gereinigt.

Im Falle von Verbindungen mit geschützten Hydroxylgruppen im Glycosylteil können die Schutzgruppen in an sich bekannter Weise abgespalten werden.

Die Phosphorylierung zur Darstellung der erfindungsgemäßen Verbindungen der Formel I kann nach jeder Methode, die für die Synthese der P-O-C-Bindung geeignet ist, durchgeführt werden.

In solchen Fällen, in denen eine Hydroxylgruppe reaktiver ist als die anderen Hydroxylgruppen im Zuckerring — dies ist z. B. die primäre Hydroxylgruppe am C-Atom 6 des Kohlenhydratteils- kann die Phosphorylierung nach konventionellen Methoden, die für selektive Phosphorylierungen z. B. aus der Nucleosid- und der Kohlenhydratchemie bekannt sind, durchgeführt werden. Solch eine Methode beinhaltet allgemein die Umsetzung eines N-Alkyl-N-(aldohexopyranosyl)-carbonsäureamids, -harnstoffs, -carbamats oder -thiocarbamats mit einem Phosphorylierungsreagenz in einem organischen Lösungsmittel.

Als geeignetes Lösungsmittel kann eine Reihe von Solventien verwendet werden, z. B. Kohlenwasserstoffe wie Hexan, Cyclohexan oder Toluol, Halogenkohlenwasserstoffe wie Dichlormethan, Chlorhexan oder Chlorbenzol, Phenole wie Phenol, Kresol, organische Säureester wie Essigester, Methylbenzoat, Nitroverbindungen wie Nitromethan, Nitroethan, Nitrobenzol, Nitrile wie Acetonitril, Malononitril, Ether wie Diethylether, Tetrahydrofuran, Dioxan, Ethylenglycoldimethylether, oder Trialkylphosphate wie Trimethylphosphat oder Triethylphosphat.

Diese Solventien können entweder allein oder als Mischung untereinander oder in Mischung mit einer organischen Base wie Pyridin, Triethylamin oder Picolin oder mit einem Salz aus einer organischen Base und einer anorganischen oder organischen Säure wie Pyridinhydrochlorid oder Pyridinium-para-Toluolsulfonat verwendet werden.

Als Phosphorylierungsreagenzien sind geeignet Phosphorhalogenide wie Phosphortrichlorid, Phosphoroxychlorid, Phenylphosphordichloridat, Diphenylphosphochloridat, Dibenzylphosphochloridat, Monalkylphosphodichloridat, Dialkylphosphochloridat oder partiell hydrolysierte Phosphoroxychloride. Als Beispiel für die Kombination von Reagenz und Solvens ist Phosphoroxychlorid und Trialkylphosphat oder Dibenzylphosphochloridat und Dichlormethan-Toluol und Pyridin besonders geeignet.

Die Reaktionstemperatur liegt zwischen —20 °C und +50 °C, bevorzugt zwischen 0 °C und Raumtemperatur, die Reaktionszeit liegt zwischen einigen Stunden und einigen Tagen.

In den Fällen, in denen eine selektive Phosphorylierung einer Hydroxylgruppe nicht durchgeführt werden kann, weil entweder eine weniger reaktive sekundäre Hydroxylgruppe des Zuckerrestes bei Vorhandensein einer reaktiveren primären Hydroxylgruppe phosphoryliert werden soll, oder aber eine

sekundäre selektiv bei Vorhandensein anderer sekundärer Hydroxylgruppen phosphoryliert werden soll, ist der eigentlichen Phosphorylierung eine Reihe von Schutzgruppenoperationen vorzuschalten, bei der selektiv die zu phosphorylierende Hydroxylgruppe am Ende der Blockierungsreaktionen frei, d. h. unsubstituiert, vorliegt. Die nicht zu phosphorylierenden Hydroxylgruppen müssen also vor der Phosphorylierung blockiert werden.

Geeignete Schutzgruppen für Zuckerderivate sind in der einschlägigen Literatur beschrieben (z. B. C. B. REESE, in : Protecting Groups in Org. Chem., 1973, p 95-p 143 ; Plenum Press). Es können alle in der Zuckerchemie verwendeten Schutzgruppen und Kombinationen daraus benutzt werden.

Geeignete Schutzgruppen sind z. B. Ester wie Acetyl, Benzoyl, Pivaloyl, p-Methoxybenzoyl, Ether wie Benzyl, p-Methoxybenzyl, Allyl, 1-Propenyl, Alkylidenverbindungen wie Ethyliden, Isopropyliden, Benzyliden, Orthoester wie 1-Methoxy-ethyliden, 1-Ethoxyethyliden, Silylether wie Trimethylsilyl, t-Butyldimethylsilyl, Organometallverbindungen wie Borsäureester oder Zinnether oder Zinnketale wie Tributylstannyl oder Dibutylstannyliden.

Die durch diese Schutzgruppen blockierten Kohlenhydratderivate werden dann an der noch freien Hydroxylgruppe in einem geeigneten Lösungsmittel phosphoryliert. Geeignete Lösungsmittel und geeignete Verfahren zur Phosphorylierung sind oben genannt.

Zur Darstellung der freien Verbindungen gemäß Formel I werden dann gegebenenfalls vorhandene Schutzgruppen nach den üblichen Methoden abgespalten.

Die Isolierung und Reinigung der erfindungsgemäßen monophosphorylierten Glycosylamide, -carbamate, -thiocarbamate oder -harnstoffe gemäß Formel I erfolgt nach konventionellen Methoden. Z. B. kann sie durchgeführt werden durch Auswahl oder Kombination der gängigen Reinigungsmethoden wie Adsorptionschromatographie an Kieselgel oder Ionenaustauscherharzen, Extraktion oder Kristallisation.

Zur Erfindung gehören auch Salze der Verbindungen der Formel I.

Dabei handelt es sich in erster Linie um üblicherweise pharmazeutisch verwendbare nichttoxische Salze wie z. B. Alkalimetall-, Erdalkalimetall- oder Ammoniumsalze.

Die Verbindungen der vorliegenden Erfindung weisen wertvolle pharmakologische Eigenschaften auf, insbesondere eine ausgeprägte antivirale und immunstimulierende Wirkung.

Es wurde gefunden, daß die Verbindungen der Erfindung die Virusvermehrung hemmen und die Interferontiter, die durch eine Virusinfektion oder durch Interferoninduktion hervorgerufen werden, signifikant erhöhen.

Diese Wirkungen können anhand der nachstehend beschriebenen Versuchsanordnung gezeigt werden.

Antivirale Wirkung

Beispiel A

Tierversuch/Kutan-Test am Meerschweinchen

Der Test wurde in Anlehnung an die von Hubler et al (J. Invest. Dermatol. 62, 92-95, (1974)) ausgearbeitete Methode durchgeführt. 500 bis 600 g schwere Meerschweinchen wurden auf der Bauchseite enthaart und mit Nembutal (15 mg/kg i.p.) narkotisiert. Vorher markierte Hautareale wurden mit einer multiplen Impflanzette (« Vaccination Gun ») infiziert. Als Virusmaterial wurde Medium von Kaninchennierenzellen benutzt, die mit Herpes simplex-Virus Typ I infiziert worden sind. Die Behandlung kann lokal, parenteral, oral, intraperitoneal oder intravenös erfolgen. Als Kontrolle dienten infizierte, nicht behandelte oder mit Placebo behandelte Tiere. Die Bewertung erfolgte nach Zahl und Größe der Herpesbläschen. Verbindungen der vorliegenden Erfindung — wie in Tab. 1 gezeigt — reduzieren Zahl und Größe der virusinduzierten Bläschen.

(Siehe Tabelle I Seite 7 f.)

Tabelle 1 Antivirale Wirkung des phosphorylierten Glycosylderivate nach Bsp. 6 auf die virusinfizierte Meerschweinchenhaut

| | | Infektionsausbruch | | | | | Abheilung | |
|---|---|---|---|---|---|---|---|---|
| Meerschweinchen | Nr. | Tag 2 | 3 | 4 | 5 | 6 | 7 | 8 |
| unbehandelte Kontrolle | 1228 | 20 | 30 | 100 | 95 | 60 | 20 | 10-20 |
| | 1229 | 20 | 70 | 90 | 85 | 55 | 20 | 10 |
| | 1230 | 10-20 | 70 | 100 | 95-100 | 50 | 20 | 10 |
| | 1231 | 15 | 30 | 80 | 70 | 60 | 20 | 10 |
| Placebo (DMSO) Kontrolle | 1232 | 20 | 30 | 100 | 100 | 60 | 20 | 10 |
| | 1233 | 10 | 20 | 50 | 50 | 60 | 20 | 10 |
| | 1234 | 80 | 70-80 | 100 | 89-90 | 50 | 20 | 10 |
| + 5 % Verbindung nach Beisp. 6 | 1241 | 10 | 20 | 5-10 | 20 | 30 | 15 | 10 |
| | 1242 | 15 | 10 | 10 | 20 | 25 | 10 | 10 |
| | 1243 | 20 | 60-70 | 10 | 30 | 35 | 15 | 10 |

100 % = große Läsionen mit z. T. konfluenten Bläschen
50 %= Läsionen mit mittelgroßen Bläschen
20 %= wenige kleine Läsionen, ohne Bläschenbildung

Interferonstimulierende Wirkung

Beispiel B

Der Test wurde in Anleitung an die von Merigan et al. (Nature 268, 67 (1977)) ausgearbeitete Methode durchgeführt. 6-8 Wochen alte CF1 Mäuse (Fa. Winkelmann, Borcken) wurden intraperitoneal mit 0,1 ml einer Lösung des Virus der lymphozytären Choriomeningitis (LCM) infiziert. Als Virusmaterial wurde ein Homogenat LCM-infizierter Mäusegehirne verwendet.

Als Kontrolle dienten infizierte, nicht behandelte oder mit Placebo behandelte Tiere.

Die Behandlung der Tiere erfolgte prophylaktisch und therapeutisch intraperitoneal (Tag — 1, 0, 1 und 2).

Das Serum der Mäuse wurde nach den in Tabelle 2 angegebenen Zeiten auf Interferonaktivität getestet. Die interferonhaltigen Seren wurden vor der Testung bei pH 2 dialysiert, um den Restvirus zu inaktivieren.

Die Bestimmung des Interferonspiegels wurde nach der Methode von Havell et. al. (Antimicrob. Agents Chemother. 2, 476 (1972)) durchgeführt.

Tabelle 2

Serum-Interferontiter (U/ml) nach Behandlung von LCM-infizierten Mäusen mit Verbindung nach Beispiel 6.

| | Tag 1 | Tag 2 | Tag 3 |
|---|---|---|---|
| Kontrolle | 0 | 800 | 2800 |
| 5 mg/kg Verb. nach Beispiel 6 | 0 | 5600 | 4000 |
| 25 mg | 3600 | 9600 | 6400 |

Verbindungen der vorliegenden Erfindung — wie in Tab. 2 gezeigt — sind in der Lage, dosisabhängig die Interferontiter zu erhöhen (bis zu 12 fache Stimulierung).

Der interferonstimulierende Effekt der genannten Verbindungen ist von einem zusätzlichen Reiz

(Virus, Interferoninduktor) abhängig, d. h. die Injektion der neuen Verbindungen hat nur zusammen mit Interferoninduktoren eine Erhöhung des Interferonspiegels zur Folge.

Die erfindungsgemäßen Verbindungen der Formel (I) weisen, wie bereits erwähnt, starke antivirale und immunstimulierende Wirkung auf.

Diese Wirkung gilt für alle Viren, die Interferon-sensitiv sind.

Als Indikationsgebiete in der Humanmedizin können für akute Infektionen beispielsweise genannt werden :

Viren der Herpesgruppe, Influenza, Rhinoviren, Enteroviren. Für persistente Infektionen : beispielsweise Viren der Herpesgruppe und Hepatitis B.

Als Indikationen in der Tiermedizin können beispielsweise aufgeführt werden :

Infektionen mit Pseudorabies-Virus (Rind, Schwein) Rhinotrachitis Virus (Rind), Rhinopneumonitis (Pferd) sowie Marek Virus (Huhn), NDV (Huhn) und Maul- und Klauenseuche (Rind, Schwein).

Die interferonstimulierende Wirkung der neuen Verbindungen eignet sich für die Stimulation der körpereigenen Abwehr, z. B. durch Beeinflußung von Makrophagen und « Natural Killer » Zellen.

Es wurde weiterhin gefunden, daß die Verbindungen der Erfindung die Antikörpersynthese des Immunsystems Antigen-spezifisch steigern und darüber hinaus die unspezifische wirtseigene Abwehr verstärken. Diese Ergebnisse wurden anhand der nachfolgenden Versuchsanordnungen erhalten.

Steigerung der primären humoralen Immunität in vitro gegen Schaferythrozyten (SE).

Es ist experimentell möglich, die Entwicklung einer humoralen Immunantwort mit heterologen roten Blutzellen durch primäre Immunisation von Maus-Milzzellen in Suspensionskulturen in vitro einzuleiten (R. I. Mishell und R. W. Dutton, J. Exp. Med. 126, 423 (1967)). Hierzu wurden Balb/c Maus-Milzzellen für 5 Tage in Gegenwart von Antigen (SE) und der Teststubstanz kultiviert. Die Zellen werden geerntet, gewaschen und zusammen mit dem Antigen und Komplement in semisolidem Agar ausplattiert und für 2 Stunden bei 37 °C inkubiert (N. K. Jerne, A. A. Nordin und C. Henryl, « Cell bound Antibodies », eds. Amos and Koprowski, Wistar Inst. Press, Philadelphia, USA, pp. 109 (1963)). Die Antigen-Sensibilisierung von Maus-Lymphozyten in der Primärkultur resultiert in der Synthese und Freisetzung von Antikörpern (AK). Die spezifischen, sezernierten Antikörper binden sich an das SE-Antigen und lysieren diese Zellen durch die Gegenwart des Komplements (Plaque-Bildung). Die Substanzen der vorliegenden Erfindung sind in der Lage, dosisabhängig im Bereich 1-100 µg/ml die Zahl der Antikörper-bildenden Zellen zu steigern.

Steigerung der primären humoralen Immunität in vivo gegen das lösliche Antigen Ovalbumin

NMRI-Mäuse wurden mit einer suboptimalen Antigendosis (1 µg/Tier, Tag 0) subcutan (s. c.) immunisiert. Bei suboptimaler Antigenstimulation wurde nur eine geringe Zahl von Lymphozyten der Tiere zur Antikörpersynthese angeregt. Die zusätzliche Behandlung der Tiere mit Verbindungen der vorliegenden Erfindung ist in der Lage, bei einer einmaligen Applikation von 30 mg/kg subcutan den Antikörpertiter im Serum der Tiere signifikat ($p \leq 0,03$) zu steigern. Die Antikörpertiterbestimmung erfolgte durch indirekte Hämagglutination am Tag 10. Der Effekt der Behandlung wird durch den geometrischen Mittelwert der $log_2$-Titer ausgedrückt.

Der immunstimulierende Effekt der erfindungsgemäßen Verbindungen ist im Gegensatz zu anderen, z. B. bakteriellen Immunstimulantien wie LPS aus Gram-negativen Bakterien Antigen-abhängig, d. h. die Substanzen bewirken überraschenderweise nur in Verbindung mit einem antigenen Reiz (hier SE oder Ovalbumin) die Induktion der Antikörpersynthese. Sie haben im Gegensatz zu den konventionellen Immunstimulantien (z. B. LPS) keine mitogenen Eigenschaften.

Verträglichkeit

Obwohl Verbindungen der beschriebenen Art ihre potenzierende Wirkung an der Maus beispielsweise bereits nach einer Einzeldosis von 10 mg/kg i. p., oder peroral entfalten, werden auch bei Applikation von 100 mg/kg keine toxischen Effekte beobachtet. Die genannten Stoffe verfügen deshalb über eine gute Verträglichkeit.

Die erfindungsgemäßen Verbindungen haben die Fähigkeit, einerseits bei Mischung mit einem Antigen dessen Immonogenität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphocyten zu aktivieren.

Die neuen Verbindungen können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die beschriebenen Verbindungen in Mischung mit verschiedensten Antigenen als Adjuvantien bei der experimentellen und industriellen Herstellung von Antiseren für Therapie und Diagnostik.

Darüber hinaus können die neuen Verbindungen auch ohne gleichzeitige Antigenzufuhr dazu benützt werden, bereits unterschwellig ablaufende Abwehrreaktionen bei Mensch und Tier zu fördern.

Die Verbindungen eignen sich demnach besonders für die Stimulation der körpereigenen Abwehr, z. B. bei chronischen und akuten Infektionen oder bei selektiven (antigenspezifischen immunologischen Defekten, sowie bei angeborenen, aber auch erworbenen allgemeinen (d. h. nicht antigenspezifischen) immunologischen Defektzuständen, wie sie im Alter, im Verlauf schwerer Primärerkrankungen und vor allem nach Therapie mit ionisierenden Strahlen oder mit immunsuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die erfindungsgemäßen Verbindungen können allein als Prophylaktikum zur Abwehr bestehender Infektionen oder in Kombination mit einer antibiotischen Therapie zur Steigerung der therapeutischen Wirkung von Antibiotika und Chemotherapeutika (z. B. Penicilline, Cephalosporine, Aminoglykoside etc.) bei infizierten Menschen und Tieren verwendet werden.

Es wurde gefunden, daß Infektionen der Maus mit pathogenen Keimen, die innerhalb von 24 bis 28 Stunden zum Tod der Versuchstiere führen, durch eine prophylaktische Behandlung — bevorzugt intraperitoneal — mit 1 bis 20 mg/kg beispielsweise der erfindungsgemäßen Verbindungen der Beispiele therapiert werden können. Dies trifft für eine ganze Reihe grampositiver (z. B. Staphylokokken) und gramnegativer (z. B. E. coli, Klebsiella, Proteus, Pseudomonas) Krankheitserreger zu.

Diese Aufzählung ist beispielhaft und keineswegs beschränkend aufzufassen. So überleben z. B. Mäuse, die mit dem pathogenen Stamm Klebsiella 63 infiziert worden waren, nach Behandlung (z. B. 18 Stunden vor Infektion) mit 1 bis 20 mg/kg der erfindungsgemäßen Verbindungen zu 40 bis 100 % diese Infektion, während von den unbehandelten Kontrolltieren nur 0 bis 30 % überlebten.

Nachweis phänotypischer Veränderungen serumresistenter in serumsensitive gramnegative Bakterienstämme.

Substanzen, die die Oberflächenstrukturen gramnegativer Bakterien modifizieren, sind in der Lage, diese Bakterien so zu verändern, daß sie empfindlicher gegenüber Wirtsabwehrmechanismen werden. Es hat sich gezeigt, daß serumresistente E. coli-Stämme, die in Gegenwart von subinhibitorischen Konzentrationen von erfindungsgemäßen Verbindungen angezüchtet wurden, phänotypisch in serumsensible Formen übergeben. Dieser Effekt konnte für die Verbindung des Beispiels 3 insgesamt mit drei verschiedenen E. coli K1-Stämmen (C10, C14, LP1674) gezeigt werden : Es erfolgte Transition von serumresistent nach serumempfindlich.

## Beispiele

### 1. N-Octadecyl-D-glucopyranosylamin

20 g Octadecylamin werden in 120 ml Ethanol gelöst und auf 70 °C erwärmt. Es werden 11 g wasserfreie D-Glucose zugesetzt. Nachdem sich eine klare Lösung gebildet hat, wird noch 15 min. bei 70 °C weitergerührt. Es wird auf 10 °C abgekühlt und 15 min. stehen gelassen. Der gebildete Kristallbrei wird abgesaugt, zweimal mit Ethanol gewaschen und im Vakuum getrocknet.

Elementaranalyse :

| | | | |
|---|---|---|---|
| ber. : | C 66,8 | H 11,4 | N 3,2 % |
| gef. : | C 67,4 | H 11,8 | N 3,7 % |

### 2. N-Glucopyranosyl-N-octadecyl-dodecansäureamid

10 g der Verbindung aus Beispiel 1 werden in 80 ml Tetrahydrofuran aufgeschlämmt und nach Zusatz von 10 g Soda tropfenweise mit 10 g Dodecansäurechlorid in 10 ml Tetrahydrofuran versetzt. Nach beendeter Reaktion (dünnschichtchromatographische Kontrolle auf Kieselgel 60 in Toluol-Isopropanol 6 : 1) wird der Feststoff abfiltriert, das Filtrat im Vakuum zum Sirup eingedampft und das Rohprodukt säulenchromatographisch auf Kieselgel 60 mit dem Elutionsmittel Toluol-Isopropanol 10 : 1 gereinigt.

$[\alpha]_D^{20} = 8°$ (c = 1,0 in Dioxan)

### 3. N-(Glucopyranosyl-6-Phosphat)-N-octadecyl-dodecansäureamid

6,1 g der Verbindung aus Beispiel 2 werden in 60 ml Dichlormethan gelöst. Zu der Lösung werden 6 g Dibenzylphosphochloridat, gelöst in 10 ml Toluol, und 3,1 ml Pyridin gegeben. Der Ansatz wird über Nacht gerührt. Danach wird filtriert, das Filtrat im Vakuum zum Sirup eingedampft und säulenchromatographisch getrennt (Laufmittel Toluol/Ethanol 10 : 1). Das erhaltene Hauptprodukt (6 g) wird in 100 ml Tetrahydrofuran und 10 ml Eisessig gelöst und in Gegenwart von 2 g Palladium-Kohle (5 %ig) hydriert. Nach beendeter Wasserstoffaufnahme wird der Katalysator abfiltriert, das Filtrat im Vakuum zum Sirup eingedampft und mehrfach mit Toluol coevaporiert.

$[\alpha]_D^{20}$ = 1,2° (c = 1,0 in Dimethylformamid)
$[\alpha]_D^{20}$ = + 8° (c = 0,065 in $H_2O$)

4. N-Dodecyl-D-galactopyranosylamin

Herstellung gemäß Beispiel 1 aus D-Galactose und Dodecylamin.

Elementaranalyse
ber. : C 62,2 H 10,7 N 4,0 %
gef. : C 62,5 H 10,2 N 4,4 %

5. N-Galactopyranosyl-N-dodecyl-octadecan-säureamid

Herstellung gemäß Beispiel 2 aus 10 g der Verbindung gemäß Beispiel 4 und 16 g Stearinsäurechlorid.

$[\alpha]_D^{20}$ = 4,4° (c = 1,0 in Dichlormethan)
Rf-Wert = 0,23 in Toluol/n-Propanol 4 : 1

6. N-(D-Galactopyranosyl-6-Phosphat)-N-dodecyl-octadecansäureamid

Herstellung gemäß Beispiel 3 aus 6 g der Verbindung aus Beispiel 5, 7,5 g Dibenzylphosphochloridat und 3,9 ml Pyridin und anschließende Hydrierung.

$[\alpha]_D^{20}$ = 11,5° (c = 0,82 in Dimethylformamid)
$[\alpha]_D^{20}$ = + 9° (c = 0,16 in $H_2O$)

7. N-(D-Lyxopyranosyl)-N-dodecyl-dodecansäureamid

10 g D-Lyxose werden in 120 ml Isopropanol und 60 ml Wasser gelöst und auf 70 °C erwärmt. Es werden 18 g Dodecylamin zugegeben. Nachdem sich eine klare Lösung gebildet hat, wird die Temperatur noch 15 min. beibehalten. Es wird auf Raumtemperatur abgekühlt und im Vakuum eingedampft. Von dem erhaltenen Sirup werden 10 g in 100 ml Tetrahydrofuran und 10 ml Methanol gelöst und mit 10 g Natriumcarbonat versetzt. Bei 0 °C werden 5,5 g Dodecansäurechlorid, gelöst in 20 ml Tetrahydrofuran, zugetropft. Nach beendeter Reaktion wird wie in Beispiel 2 beschrieben aufgearbeitet und das Produkt gereinigt.

$[\alpha]_D^{20}$ = 5,3° (c = 1,1 in Tetrahydrofuran)

Elementaranalyse :
ber. : C 69,7 H 11,5 N 2,8 %
gef. : C 69,6 H 11,7 N 2,7 %

8. N-(D-Lyxopyranosyl-4-phosphat)-N-dodecyldodecansäureamid

5 g der Verbindung aus Beispiel 7 werden in 30 ml Tetrahydrofuran gelöst. Es werden 1 ml 2,2-Dimethoxypropan und 10 mg para-Toluolsulfonsäure zugesetzt und 30 min. auf 70 °C erwärmt. Nach dem Abkühlen auf Raumtemperatur wird mit Ionenaustauscher MP 500 (OH-Form) neutralisiert und mehrfach mit Toluol im Vakuum eingedampft. Der erhaltene Sirup wird in 50 ml Dichlormethan gelöst und mit 6 g Dibenzylphosphochloridat und 3,1 ml Pyridin versetzt und über Nacht gerührt. Es wird vom gebildeten Feststoff abfiltriert und das Filtrat zum Sirup eingeengt. Der Sirup wird säulenchromatographisch gereinigt (Laufmittel Toluol/Aceton 10 : 1). Das erhaltene Hauptprodukt wird in 30 ml Dichlormethan gelöst und bei 0 °C mit 2 ml Trifluoressigsäure/Wasser (99 : 1) versetzt. Nach 10 min wird mit 30 ml Toluol verdünnt und im Vakuum eingeengt. Der Rückstand wird dreimal in Toluol aufgenommen und im Vakuum eingeengt. Der erhaltene Sirup wird in 80 ml Tetrahydrofuran und 10 ml Eisessig gelöst und in Gegenwart von 2 g Palladiumkohle (5 %ig) hydriert. Nach beendeter Wasserstoffaufnahme wird der Katalysator abfiltriert, das Filtrat im Vakuum zum Sirup eingedampft und mehrfach mit Toluol coevaporiert.

$[\alpha]_D^{20}$ = 2,7° (c = 0,95 in Tetrahydrofuran)

9. N-Octadecyl-N-(D-glucopyranosyl)-decylurethan

9 g der Verbindung aus Beispiel 1 werden in 160 ml Tetrahydrofuran und 40 ml Ethanol aufgeschlämmt und mit 9 g Natriumcarbonat versetzt. Zu dieser Suspension werden 5 g Chlorameisensäuredecylester, gelöst in 40 ml Tetrahydrofuran, während 20 min. zugetropft. Nach beendeter Reaktion wird der Ansatz filtriert, der Filterrückstand mit Tetrahydrofuran nachgewaschen. Das Filtrat wird mit den

Waschlösungen vereinigt und im Vakuum eingedampft. Der erhaltene Sirup wird chromatographisch gereinigt (Laufmittel Dichlormethan/Methanol, 20 : 1).

Rf-Wert : 0,37 in $CH_2Cl_2/CH_3OH$ 10 : 1

Elementaranalyse
ber. : C 68,3 H 11,3 N 2,3 %
gef. : C 68,4 H 11,6 N 2,4 %

10. N-Octadecyl-N-(D-glucopyranosyl-6-Phosphat)-decylurethan

Herstellung gemäß Beispiel 3 aus 6 g der Verbindung aus Beispiel 9, 7,5 g Dibenzylphosphochloridat und 3,9 ml Pyridin und anschließende Hydrierung.

$[\alpha]_D^{20}$ = 1,9° (c = 0,75 in Dimethylformamid)

11. N-Octadecyl-N-(D-glucopyranosyl)-N'-dodecylharnstoff

9 g der Verbindung aus Beispiel 1 werden in 160 ml Tetrahydrofuran und 40 ml Ethanol aufgeschlämmt. Zu dieser Suspension werden 4,3 g Dodecylisocyanat, gelöst in 20 ml Tetrahydrofuran, während 20 min getropft. Nach beendeter Reaktion wird i. Vak. eingedampft und der erhaltene Sirup säulenchromatographisch gereinigt (Laufmittel Dichlormethan/Methanol, 15 : 1)

Rf-Wert : 0,33 in $CH_2Cl_2/CH_3OH$ 10 : 1

$[\alpha]_D^{20}$ = 7,4° (c = 1,04 in Dioxan)

12. N-Octadecyl-N-(D-glucopyranosyl-6-Phosphat)-N'-dodecylharnstoff

Herstellung gemäß Beispiel 3 aus 6 g der Verbindung aus Beispiel 11, 7,5 g Dibenzylphosphochloridat und 3,9 ml Pyridin und anschließende Hydrierung.

$[\alpha]_D^{20}$ = 1,0° (c = 2,10 in Dimethylformamid)

13. N-Octadecyl-N-(D-glucopyranosyl-4-Phosphat)-dodecansäureamid

10 g der Verbindung aus Beispiel 2 werden in 100 ml Tetrahydrofuran gelöst, mit 3,5 g Benzaldehyddimethylacetal und 10 mg para-Toluolsulfonsäure versetzt und 1 h auf 70 °C erwärmt. Nach dem Abkühlen auf Raumtemperatur wird mit Ionenaustauscher MP 500 (OH-Form) neutralisiert und im Hochvakuum eingeengt. Der erhaltene Sirup wird in 100 ml Tetrahydrofuran aufgenommen und nach Zugabe von 1,5 g Natriumhydrid und 3,5 ml Benzylbromid 1 h auf 40 °C erwärmt. Nach dem Abkühlen auf Raumtemperatur werden 20 ml Methanol zugesetzt und 1 h nachgerührt. Der Ansatz wird vorsichtig mit 10 ml Wasser versetzt und im Hochvakuum eingeengt. Der erhaltene Sirup wird in 100 ml Dichlormethan aufgenommen, zweimal mit je 20 ml Wasser extrahiert, über Magnesiumsulfat getrocknet, eingeengt im Vakuum und säulenchromatographisch gereinigt (Laufmittel Toluol/Essigester 20 : 1).

Der erhaltene Sirup wird in 30 ml Tetrahydrofuran gelöst und nach Zugabe von 30 ml Eisessig/Wasser 10 : 1 1 h auf 70 °C erwärmt, auf Raumtemperatur abgekühlt und mehrfach mit Toluol im Hochvakuum eingedampft. Der erhaltene, chromatographisch einheitliche Sirup (7,8 g) wird in 100 ml Tetrahydrofuran gelöst, 30 min. mit 400 mg Natriumhydrid bei 50 °C gerührt, auf 0 °C gekühlt und mit 1,8 g Benzylbromid versetzt. Nach 3 h wird auf Raumtemperatur erwärmt und über Nacht weitergerührt. Nach Zugabe von 5 ml Methanol wird 1 h nachgerührt, dann wird vorsichtig mit Wasser versetzt und eingedampft. Der erhaltene Rückstand wird in Dichlormethan und Wasser aufgenommen und die organische Phase mit Wasser extrahiert, getrocknet und eingeengt im Hochvakuum. Der Rückstand wird in 10 ml Tetrahydrofuran aufgenommen und die Lösung mit 5,5 g Dibenzylphosphochloridat und 2,8 ml Pyridin über Nacht gerührt. Es wird filtriert, das Filtrat im Vakuum zum Sirup eingedampft und säulenchromatographisch gereinigt (Laufmittel Toluol/Essigester 15 : 1).

Das erhaltene Hauptprodukt (3,9 g) wird in 80 ml Tetrahydrofuran und 10 ml Eisessig gelöst und in Gegenwart von 2 g Palladiumkohle hydriert. Nach beendeter Wasserstoffaufnahme wird der Katalysator abfiltriert, das Filtrat im Vakuum zum Sirup eingedampft und mehrfach mit Toluol coevaporiert.

$[\alpha]_D^{20}$ = 1,9° (c = 2,0 in Dimethylformamid)

Analog zu den Beispielen 3 und 6 wurden synthetisiert durch Umsetzung der Aldosen mit Alkylaminen zum Aldosylamin, dessen Acylierung mit Fettsäurechloriden zum Aldosylamid und anschließende selektive Phosphorylierung und Hydrierung :

14. N-(Dodecyl)-N-(D-Galactopyranosyl-6-Phosphat)-hexadecansäureamid

$[\alpha]_D^{20}$ = 12° (c = 1,24 in Dimethylformamid)

15. N-(Dodecyl)-N-(D-Galactopyranosyl-6-Phosphat)-tetradecansäureamid

16. N-(Tetradecyl)-N-(D-Galactopyranosyl-6-Phosphat)-octadecansäureamid

17. N-(Tetradecyl)-N-(D-Galactopyranosyl-6-Phosphat)-ölsäureamid

18. N-(Tetradecyl)-N-(D-Galactopyranosyl-6-Phosphat)-hexadecansäureamid

19. N-(Tetradecyl)-N-(D-Galactopyranosyl-6-Phosphat)-tetradecansäureamid

20. N-(Hexadecyl)-N-(D-Galactopyranosyl-6-Phosphat)-dodecansäureamid

21. N-(Octadecyl)-N-(D-Galactopyranosyl-6-Phosphat)-dodecansäureamid

Di-Natriumsalz : $[\alpha]_D^{20}$ = —0,1° (c = 0,43 in $H_2O$)

22. N-(Octadecyl)-N-(D-Galactopyranosyl-6-Phosphat)-tetradecansäureamid

23. N-(Octadecyl)-N-(D-Galactopyranosyl-6-Phosphat)-octadecansäureamid

24. N-(Octadecyl)-N-(D-Glucopyranosyl-6-Phosphat)-tetradecansäureamid

Di-Natriumsalz : $[\alpha]_D^{20}$ = — 1° (c = 0,35 in $H_2O$)

25. N-(Octadecyl)-N-(D-Glucopyranosyl-6-Phosphat)-octadecansäureamid

Di-Natriumsalz : $[\alpha]_D^{20}$ = + 0,4° (c = 0,1 in $H_2O$)

26. N-(Octadecyl)-N-(D-Glucopyranosyl-6-Phosphat)-ölsäureamid

27. N-(Tetradecyl)-N-(D-Glucopyranosyl-6-Phosphat)-octadecansäureamid

Di-Natriumsalz : $[\alpha]_D^{20}$ = + 6° (c = 0,07 in $H_2O$)

28. N-(Octadecyl)-N-(D-Mannopyranosyl-6-Phosphat)-tetradecansäureamid

Di-Natriumsalz : $[\alpha]_D^{20}$ = + 4° (c = 0,25 in $H_2O$)

29. N-(Octadecyl)-N-(D-Mannopyranosyl-6-Phosphat)-octadecansäureamid

30. N-(Tetradecyl)-N-(D-Mannopyranosyl-6-Phosphat)-octadecansäureamid

31. N-(Dodecyl)-N-(D-Mannopyranosyl-6-Phosphat)-octadecansäureamid

**Patentansprüche** (für die Vertragsstaaten : BE, CH, DE, FR, GB, IT, LI, NL, SE)

1. Verbindungen der allgemeinen Formel

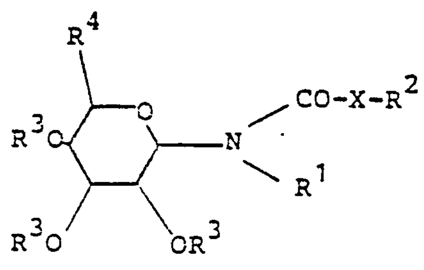

in der

$R^1$ einen gegebenenfalls substituierten, geradkettigen oder verzweigten, gesättigten oder ein- oder

mehrfach ungesättigten Alkylrest, einen gegebenenfalls substituierten, gesättigten oder ein- oder mehrfach ungesättigten Cycloalkylrest, einen gegebenenfalls substituierten Arylrest oder Aralkylrest darstellt,

X für $CH_2$, O, S, NH oder N-Alkyl mit bis zu 20 C-Atomen steht,

$R^2$ die Bedeutung von $R^1$ hat und für Wasserstoff stehen kann, wenn X die Bedeutung $CH_2$ hat,

$R^3$ unabhängig voneinander Wasserstoff, einen Acylrest oder Alkylrest mit jeweils bis zu 20 C-Atomen, einen Silylrest und/oder einen Phosphorsäure- oder Phosphorsäureesterrest dardarstellt und

$R^4$ Wasserstoff, Methyl oder $—CH_2—OR^3$ bedeutet

mit der Maßgabe, daß immer einer der Reste $R^3$ für einen Phosphorsäure- oder Phosphorsäureesterrest steht.

2. Verbindungen nach Anspruch 1, dadurch gekennzeichnet, daß $R^1$ einen Alkylrest oder Alkenylrest mit 10-20 C-Atomen darstellt.

3. Verbindungen nach den Ansprüchen 1 oder 2, dadurch gekennzeichnet, daß der Alkyl- oder Acylrest in der Bedeutung von $R^3$ 1-10 C-Atome hat.

4. Verbindungen nach einem der Ansprüche 1-3, dadurch gekennzeichnet, daß der Phosphorsäurerest ein Rest der Formel $—PO(OH)_2$ und der Phosphorsäureesterrest ein Rest der Formel

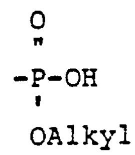

oder

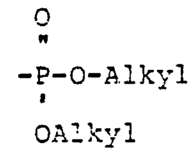

ist, in denen Alkyl einen geradkettigen oder verzweigten Alkylrest mit 1-20 C-Atomen darstellt.

5. Verbindungen nach Anspruch 4, dadurch gekennzeichnet, daß der Alkylrest des Phosphorsäureesterrestes 1-5 C-Atome hat.

6. Verfahren zur Herstellung der Verbindungen gemäß Anspruch 1, dadurch gekennzeichnet, daß man eine Pentose oder Hexose mit einem Amin der Formel $R^1$-$NH_2$, in der $R^1$ die in Anspruch 1 genannte Bedeutung hat, zu einem Glycosylamin der Formel II

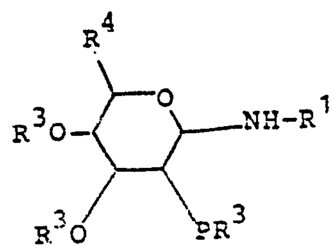

(II)

worin $R^3$ und $R^4$ die in Anspruch 1 angegebene Bedeutung haben, wobei jedoch nicht einer der Reste $R^3$ einen Phosphorsäure- oder Phosphorsäureesterrest darstellen muß, umsetzt, die Verbindung der Formel II unter Einführung des Restes $—CO—X—R^2$ mit $R^2$ in der in Anspruch 1 angegebenen Bedeutung acyliert und gegebenenfalls, wenn nicht schon einer der Reste $R^3$ in Formel II einen Phosphorsäure- oder Phosphorsäureesterrest darstellt, eine Phosphorylierung anschließt.

7. Arzneimittel enthaltend mindestens eine Verbindung gemäß einem der Ansprüche 1-5.

8. Verwendung der Verbindung gemäß den Ansprüchen 1-5 zur Herstellung von Arzneimitteln für die Bekämpfung von Krankheiten.

9. Verwendung der Verbindungen gemäß den Ansprüchen 1-5 zur Herstellung von Arzneimitteln für die Bekämpfung viraler und bakterieller Infektionen.

10. Verwendung der Verbindungen gemäß den Ansprüchen 1-5 zur Herstellung von Arzneimitteln für die Immunstimulation.

**Patentansprüche** (für den Vertragsstaat AT)

1. Verfahren zur Herstellung der Verbindungen der allgemeinen Formel (I)

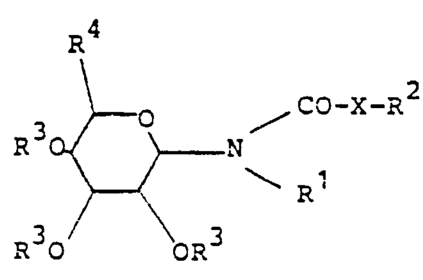

in der

$R^1$ einen gegebenenfalls substituierten, geradkettigen oder verzweigten, gesättigten oder ein- oder mehrfach ungesättigten Alkylrest, einen gegebenenfalls substituierten, gesättigten oder ein- oder mehrfach ungesättigten Cycloalkylrest, einen gegebenenfalls substituierten Arylrest oder Aralkylrest darstellt,

X für $CH_2$, O, S, NH oder N-Alkyl mit bis zu 20 C-Atomen steht,

$R^2$ die Bedeutung von $R^1$ hat und für Wasserstoff stehen kann, wenn X die Bedeutung $CH_2$ hat,

$R^3$ unabhängig voneinander Wasserstoff, einen Acylrest oder Alkylrest mit jeweils bis zu 20 C-Atomen, einen Silylrest und/oder einen Phosphorsäure- oder Phosphorsäureesterrest darstellt und

$R^4$ Wasserstoff, Methyl oder $—CH_2—OR^3$ bedeutet

mit der Maßgabe, daß immer einer der Reste $R^3$ für einen Phosphorsäure- oder Phosphorsäureesterrest steht,

dadurch gekennzeichnet, daß man eine Pentose oder Hexose mit einem Amin der Formel

$$R^1—NH_2$$

in der $R^1$ die oben angegebene Bedeutung hat, zu einem Glycosylamin der Formel (II)

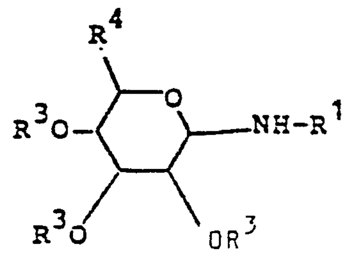

worin $R^3$ und $R^4$ die oben angegebene Bedeutung haben, wobei jedoch nicht einer der Reste $R^3$ einen Phosphorsäure- oder Phosphorsäureesterrest darstellen darf, umsetzt, die Verbindung der Formel (II) unter Einführung des Restes $—CO—X—R^2$ mit $R^2$ in der oben angegebenen Bedeutung acyliert und gegebenenfalls, wenn nicht schon einer der Reste $R^3$ in Formel (II) einen Phosphorsäure- oder Phosphorsäureesterrest darstellt, eine Phosphorylierung anschließt.

2. Verfahren nach Anspruch 1, dadurch gekennzeichnet, daß man in einem ersten Verfahrensschritt einen unblockierten Zucker aus der Aldopentose- oder Aldohexosereihe mit einem Amin der Formel $R^1—NH_2$, in der $R^1$ die in Anspruch 1 angegebene Bedeutung hat, zur Reaktion bringt, wobei unter Wasserabspaltung ein Glycosylamin der Formel (II) entsteht, mit der Maßgabe, daß für diesen Fall $R^3$ für Wasserstoff und $R^4$ entweder für Wasserstoff, Methyl oder Hydroxymethyl steht.

3. Verfahren nach Anspruch 1, dadurch gekennzeichnet, daß man die Pentose oder Hexose mit einem flüssigen Amin der Formel $R^1—NH_2$, in der $R^1$ die in Anspruch 1 angegebene Bedeutung hat, in Abwesenheit von Lösungsmitteln bei Temperaturen zwischen 0 und 100 °C, gegebenenfalls in Gegenwart katalytischer Mengen Mineralsäuren, umsetzt.

4. Verfahren nach Anspruch 3, dadurch gekennzeichnet, daß man die Mineralsäuren in Mengen von 0,001 bis 0,05 Äquivalenten, bezogen auf den Aminanteil, einsetzt.

5. Verfahren nach Anspruch 1, dadurch gekennzeichnet, daß man die Umsetzung in Gegenwart eines Lösungsmittels durchführt und dabei Temperaturen von —10 °C bis 120 °C anwendet.

6. Verfahren nach Anspruch 1, dadurch gekennzeichnet, daß man die Reaktion in Methanol, Ethanol, 1-Propanol, 2-Propanol, Tetrahydrofuran, Dioxan oder Dimethylformamid, gegebenenfalls unter Zusatz von Wasser, durchführt.

7. Verfahren nach Anspruch 1, dadurch gekennzeichnet, daß man für den Fall, daß X in der Formel (I) eine Methylengruppe darstellt, die entsprechenden Glycosylamine der Formel (II) mit 1-10 Äquivalenten eines Carbonsäurederivats der Formel $R^2—CH_2—CO—Y$, in der $R^2$ die in Anspruch 1 angegebene Bedeutung hat und Y für Halogen oder eine bei Amidierungsreaktionen übliche Abgangsgruppe steht, umsetzt.

8. Verfahren nach Anspruch 1, dadurch gekennzeichnet, daß man für den Fall, daß X in der Formel (I) ein Sauerstoffatom darstellt, ein Glycosylamin der Formel (II) mit 1-5 Äquivalenten eines Halogenkohlensäureesters $Y—CO—O—R^2$, wobei Y für Halogen steht und $R^2$ die in Anspruch 1 beschriebene Bedeutung hat, umsetzt.

9. Verfahren nach Anspruch 1, dadurch gekennzeichnet, daß man für den Fall, daß X in der Formel (I) ein Schwefelatom darstellt, ein Glycosylamin der Formel (II) mit einem Thiokohlensäurechlorid-S-ester $R^2—S—CO—Y$, wobei Y für Halogen steht und $R^2$ die in Anspruch 1 angegebene Bedeutung hat, umsetzt.

10. Verfahren nach Anspruch 1, dadurch gekennzeichnet, daß man für den Fall, daß X in Formel (I) eine NH-Gruppe bedeutet, ein Glycosylamin der Formel (II) mit 1-5 Äquivalenten eines Isocyanats $R^2—NCO$ mit der in Anspruch 1 angegebenen Bezeichnung von $R^2$ umsetzt, oder daß man für den Fall, daß X in Formel (I) eine NH-Gruppe oder eine N-Alkyl-Gruppe bedeutet, ein Carbamat, das man durch

Reaktion des Amins gemäß Formel (II) mit einem aromatischen Halogenameisensäureester erhält, mit 1-10 Äquivalenten eines primären Amins $R^2$—$NH_2$ oder eines sekundäuren Amins $R^2$—NH-Alkyl mit den in Anspruch 1 beschriebenen Bedeutungen für $R^2$ zur Reaktion bringt.

**Claims** (for the Contracting States : BE, CH, DE, FR, GB, IT, LI, NL, SE)

1. Compounds of the general formula

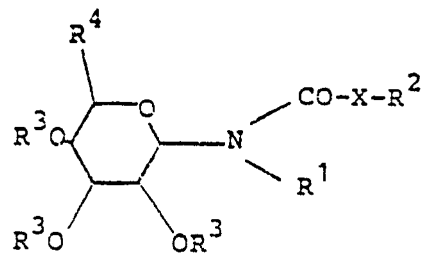

in which

$R^1$ represents an optionally substituted, straightchain or branched, saturated or monounsaturated or polyunsaturated alkyl radical, an optionally substituted, saturated or monounsaturated or polyunsaturated cycloalkyl radical or an optionally substituted aryl radical or aralkyl radical,

X represents $CH_2$, O, S, NH or N-alkyl with up to 20 C atoms,

$R^2$ has the meaning of $R^1$, and can represent hydrogen if X denotes $CH_2$,

the radicals $R^3$ independently of the another represent hydrogen, an acyl radical or alkyl radical with in each case up to 20 C atoms, a silyl radical and/or a phosphoric acid or phosphoric acid ester radical and

$R^4$ denotes hydrogen, methyl or —$CH_2$—$OR^3$,

with the proviso that one of the radicals $R^3$ always represents a phosphoric acid or phosphoric acid ester radical.

2. Compounds according to Claim 1, characterized in that $R^1$ represents an alkyl radical or alkenyl radical with 10-20 C atoms.

3. Compounds according to Claims 1 or 2, characterized in that the alkyl or acyl radical $R^3$ has 1-10 C atoms.

4. Compounds according to one of Claims 1-3, characterized in that the phosphoric acid radical is a radical of the formula —$PO(OH)_2$, and the phosphoric acid ester radical is a radical of the formula

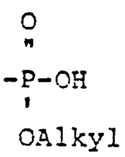

or

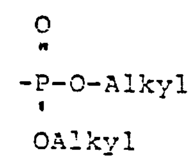

in which alkyl represents a straight-chain or branched alkyl radical with 1-20 C atoms.

5. Compounds according to Claim 4, characterized in that the alkyl radical of the phosphoric acid ester radical has 1-5 C atoms.

6. Process for the preparation of the compounds according to Claim 1, characterized in that a pentose or hexose is reacted with an amine of the formula $R^1$—$NH_2$, in which $R^1$ has the meaning given in Claim 1, to give a glycosylamine of the formula II

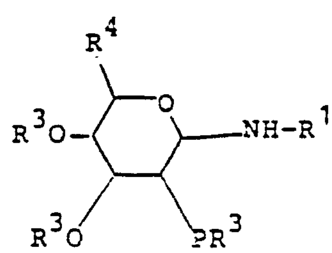

(II)

wherein $R^3$ and $R^4$ have the meaning given in Claim 1, but where it is not necessary for one of the radicals $R^3$ to represent a phosphoric acid or phosphoric acid ester radical, the compound of the formula II is acylated with insertion of the radical —CO—X—$R^2$, where $R^2$ has the meaning given in Claim 1, and, if appropriate, if one of the radicals $R^3$ in formula II does not already represent a phosphoric acid or phosphoric acid ester radical, phosphorylation is subsequently carried out.

7. Medicaments containing at least one compound according to one of Claims 1-5.

8. Use of the compounds according to Claims 1-5 for the preparation of medicaments for combating diseases.

9. Use of the compounds according to Claims 1-5 for the preparation of medicaments for combating viral and bacterial infections.

10. Use of the compounds according to Claims 1-5 for the preparation of medicaments for immunostimulation.

**Claims** (for the Contracting State AT)

1. Process for the preparation of the compounds of the general formula (I)

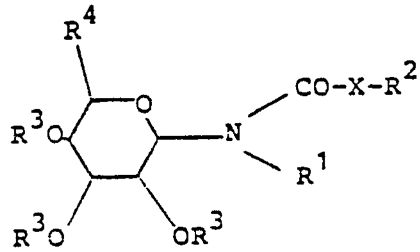

in which

$R^1$ represents an optionally substituted, straightchain or branched, saturated or monounsaturated or polyunsaturated alkyl radical, an optionally substituted, saturated or monounsaturated or polyunsaturated cycloalkyl radical or an optionally substituted aryl radical or aralkyl radical,

X represents $CH_2$, O, S, NH or N-alkyl with up to 20 C atoms,

$R^2$ has the meaning of $R^1$, and can represent hydrogen if X denotes $CH_2$,

the radicals $R^3$ independently of one another represent hydrogen, an acyl radical or alkyl radical with in each case up to 20 C atoms, a silyl radical and/or a phosphoric acid or phosphoric acid ester radical and

$R^4$ denotes hydrogen, methyl or $—CH_2—OR^3$,

with the proviso that one of the radicals $R^3$ always represents a phosphoric acid or phosphoric acid ester radical,

characterized in that a pentose or hexose is reacted with an amine of the formula

$$R^1—NH_2$$

in which $R^1$ has the abovementioned meaning, to give a glycosylamine of the formula (II)

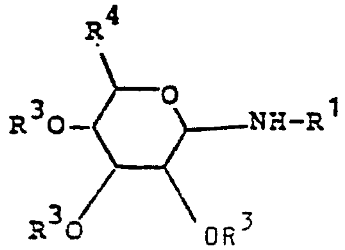

wherein $R^3$ and $R^4$ have the abovementioned meaning, but where one of the radicals $R^3$ must not represent a phosphoric acid or phosphoric acid ester radical, the compound of the formula (II) is acylated with insertion of the radical $—CO—X—R^2$, where $R^2$ has the abovementioned meaning, and, if appropriate, if one of the radicals $R^3$ in formula (II) does not already represent a phosphoric acid or phosphoric acid ester radical, phosphorylation is subsequently carried out.

2. Process according to Claim 1, characterized in that, in a first process step, a non-blocked sugar of the aldopentose or aldohexose series is reacted with an amine of the formula $R^1—NH_2$, in which $R^1$ has the meaning given in Claim 1, a glycosylamine of the formula (II) being formed, with water being split off, with the proviso that in this case $R^3$ represents hydrogen and $R^4$ represents either hydrogen, methyl or hydroxymethyl.

3. Process according to Claim 1, characterized in that the pentose or hexose is reacted with a liquid amine of the formula $R^1—NH_2$, in which $R^1$ has the meaning given in Claim 1, in the absence of solvents at temperatures between 0 and 100 °C, if appropriate in the presence of catalytic amounts of mineral acids.

4. Process according to Claim 3, characterized in that the mineral acids are used in amounts of 0.001 to 0.05 equivalents, based on the amine content.

5. Process according to Claim 1, characterized in that the reaction is carried out in the presence of a solvent and temperatures of — 10 °C to 120 °C are thereby applied.

6. Process according to Claim 1, characterized in that the reaction is carried out in methanol, ethanol, 1-propanol, 2-propanol, tetrahydrofuran, dioxane or dimethylformamide, if appropriate with the addition of water.

7. Process according to Claim 1, characterized in that, if X in formula (I) represents a methylene group, the corresponding glycosylamines of the formula (II) are reacted with 1-10 equivalents of a carboxylic acid derivative of the formula $R^2—CH_2—CO—Y$, in which $R^2$ has the meaning given in Claim 1 and Y represents halogen or a leaving group customary in amidation reactions.

8. Process according to Claim 1, characterized in that, if X in formula (I) represents an oxygen atom, a glycosylamine of the formula (II) is reacted with 1-5 equivalents of a halogenocarbonic acid ester $Y—CO—O—R^2$, wherein Y represents halogen and $R^2$ has the meaning described in Claim 1.

9. Process according to Claim 1, characterized in that, if X in formula (I) represents a sulphur atom, a glycosylamine of the formula (II) is reacted with a thiocarbonic acid chloride S-ester $R^2—S—CO—Y$, wherein Y represents halogen and $R^2$ has the meaning given in Claim 1.

10. Process according to Claim 1, characterized in that, if X in formula (I) denotes an NH group, a glycosylamine of the formula (II) is reacted with 1-5 equivalents of an isocyanate $R^2—NCO$, with the designation of $R^2$ given in Claim 1, or in that, if X in formula (I) denotes an NH group or an N-alkyl group, a carbamate, which is obtained by reaction of the amine according to formula (II) with an aromatic halogenoformic acid ester, is reacted with 1-10 equivalents of a primary amine $R^2—NH_2$ or of a secondary amine $R^2$—NH-alkyl, with the meanings for $R^2$ described in Claim 1.

**Revendications** (pour les Etats contractants : BE, CH, DE, FR, GB, IT, LI, NL, SE)

1. Composés de formule générale :

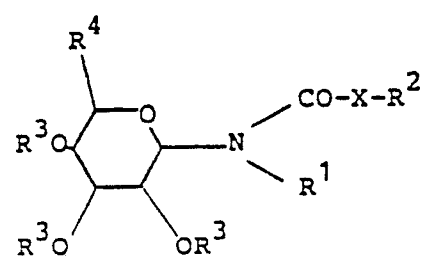

dans laquelle

$R^1$ représente : un reste alkyle linéaire ou ramifié, saturé ou insaturé une ou plusieurs fois, éventuellement substitué, ou un reste cycloalkyle saturé ou insaturé une ou plusieurs fois, éventuellement substitué, un reste aryle ou aralkyle éventuellement substitué,

X représente $CH_2$, O, S, NH ou N-alkyle ayant jusqu'à 20 atomes de carbone,

$R^2$ a le sens de $R^1$ et peut représenter un atome d'hydrogène quand X représente $CH_2$,

les $R^3$ représentent chacun, indépendamment l'un de l'autre, un atome d'hydrogène, un reste acyle ou un reste alkyle ayant chacun jusqu'à 20 atomes de carbone, un reste silyle et/ou un reste acide phosphorique ou ester d'acide phosphorique, et

$R^4$ représente un atome d'hydrogène, un groupe méthyle ou $—CH_2—OR^3$,

à la condition que l'un des restes $R^3$ représente toujours un reste acide phosphorique ou ester d'acide phosphorique.

2. Composés selon la revendication 1, caractérisés en ce que $R^1$ représente un reste alkyle ou un reste alcényle ayant 10 à 20 atomes de carbone.

3. Composés selon les revendications 1 ou 2, caractérisés en ce que le reste alkyle ou acyle représenté par $R^3$ comporte 1 à 10 atomes de carbone.

4. Composés selon l'une des revendications 1 à 3, caractérisés en ce que le reste acide phosphorique est un reste de formule $—PO(OH)_2$, et le reste ester d'acide phosphorique est un reste de formule :

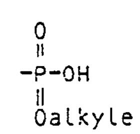

ou

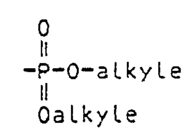

dans lesquelles « alkyle » représente un reste alkyle, linéaire ou ramifié, ayant 1 à 20 atomes de carbone.

5. Composés selon la revendication 4, caractérisés en ce que le reste alkyle du reste ester d'acide phosphorique comporte 1 à 5 atomes de carbone.

6. Procédé pour préparer les composés selon la revendication 1, caractérisé en ce qu'on fait réagir un pentose ou un hexose avec une amine de formule $R^1—NH_2$ (dans laquelle $R^1$ a le sens indiqué à la revendication 1) pour obtenir une glycosylamine de formule II

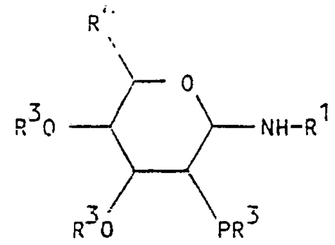

(II)

dans laquelle $R^3$ et $R^4$ ont le sens indiqué à la revendication 1, mais l'un des restes $R^3$ ne devant pas cependant pas obligatoirement représenter un reste acide phosphorique ou ester d'acide phosphorique ; on acyle le composé de formule II en y introduisant le reste $—CO—X—R^2$, dans lequel $R^2$ a le sens indiqué à la revendication 1, et éventuellement, lorsque l'un des restes $R^3$ de la formule II ne représente pas déjà un reste acide phosphorique ou ester d'acide phosphorique, on effectue ensuite une phosphorylation.

7. Médicament contenant au moins un composé selon l'une des revendications 1 à 5.

8. Utilisation des composés selon les revendications 1 à 5 pour préparer des médicaments destinés à combattre des maladies.

9. Utilisation des composés selon les revendications 1 à 5 pour préparer des médicaments destinés à combattre des infections virales et bactériennes.

10. Utilisation des composés selon les revendications 1 à 5 pour préparer des médicaments pour stimuler les processus immunitaires.

**Revendications** (pour l'Etat contractant AT)

1. Procédé pour préparer les composés de formule générale (I) :

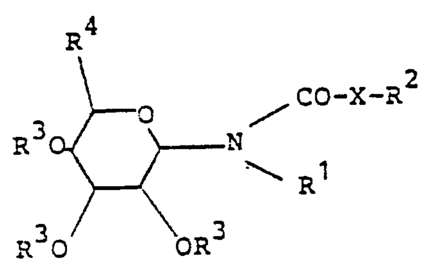

dans laquelle

$R^1$ représente : un reste alkyle linéaire ou ramifié, saturé ou insaturé une ou plusieurs fois, éventuellement substitué, ou un reste cycloalkyle saturé ou insaturé une ou plusieurs fois, éventuellement substitué, un reste aryle ou aralkyle éventuellement substitué,

X représente $CH_2$, O, S, NH ou N-alkyle ayant jusqu'à 20 atomes de carbone,

$R^2$ a le sens de $R^1$ et peut représenter un atome d'hydrogène quand X représente $CH_2$,

les $R^3$ représentent chacun, indépendamment l'un de l'autre, un atome d'hydrogène, un reste acyle ou un reste alkyle ayant chacun jusqu'à 20 atomes de carbone, un reste silyle et/ou un reste acide phosphorique ou ester d'acide phosphorique, et

$R^4$ représente un atome d'hydrogène, un groupe méthyle ou $—CH_2—OR^3$,

à la condition que l'un des restes $R^3$ représente toujours un reste acide phosphorique ou ester d'acide phosphorique,

procédé caractérisé en ce qu'on fait réagir un pentose ou hexose avec une amine de formule :

$$R^1—NH_2,$$

dans laquelle $R^1$ a le sens indiqué ci-dessus, pour obtenir une glycosylamine de formule (II)

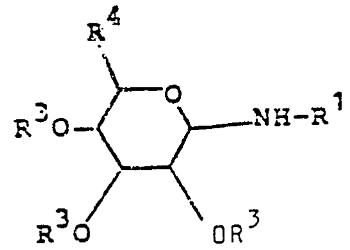

dans laquelle $R^3$ et $R^4$ ont le sens indiqué ci-dessus, mais l'un des restes $R^3$ ne devant pas obligatoirement représenter un reste acide phosphorique ou ester d'acide phosphorique ; on acyle le composé de formule (II) en y introduisant le reste $—CO—X—R^2$, où $R^2$ a le sens indiqué ci-dessus, et éventuellement, quand l'un des restes $R^3$ de la formule (II) ne représente pas déjà un reste acide phosphorique ou ester d'acide phosphorique, on effectue ensuite une phosphorylation.

2. Procédé selon la revendication 1, caractérisé en ce que, dans une première étape, on fait réagir un sucre non bloqué, de la série des aldopentoses ou des aldohexoses, avec une amine de formule $R^1—NH_2$, dans laquelle $R^1$ a le sens indiqué à la revendication 1, ce qui donne, avec séparation d'eau, une glycosylamine de formule (II), à la condition que, dans ce cas, $R^3$ représente un atome d'hydrogène et $R^4$ représente un atome d'hydrogène, un groupe méthyle ou hydroxyméthyle.

3. Procédé selon la revendication 1, caractérisé en ce qu'on fait réagir le pentose ou l'hexose avec une amine liquide de formule $R^1—NH_2$, dans laquelle $R^1$ a le sens indiqué à la revendication 1, en l'absence de solvants, à des températures comprises entre 0 et 100 °C, éventuellement en présence de quantités catalytiques d'acides minéraux.

4. Procédé selon la revendication 3, caractérisé en ce qu'on utilise les acides minéraux en des quantités de 0,001 à 0,05 équivalent, par rapport à la partie amine.

5. Procédé selon la revendication 1, caractérisé en ce qu'on conduit la réaction en présence d'un solvant et l'on utilise pour cela des températures comprises entre — 10 °C et 120 °C.

6. Procédé selon la revendication 1, caractérisé en ce qu'on conduit la réaction dans du méthanol, de l'éthanol, du 1-propanol, du 2-propanol, du tétrahydrofuranne, du dioxanne ou du diméthylformamide, éventuellement avec addition d'eau.

7. Procédé selon la revendication 1, caractérisé en ce que, si X dans la formule (I) représente un groupe méthylène, on fait réagir la glycosylamine correspondante, de formule (II), avec 1 à 10 équivalents d'un dérivé d'acide carboxylique de formule $R^2—CH_2—CO—Y$, dans laquelle $R^2$ a le sens indiqué à la revendication 1 et Y représente un halogène ou un groupe partant, usuel dans les réactions d'amidation.

8. Procédé selon la revendication 1, caractérisé en ce que, si, dans la formule (I), X représente un atome d'oxygène, on fait réagir une glycosylamine de formule (II) avec 1 à 5 équivalents d'un ester d'acide halogénocarbonique $Y—CO—O—R^2$, dans lequel Y représente un halogène et $R^2$ a le sens décrit à la revendication 1.

9. Procédé selon la revendication 1, caractérisé en ce que, si, dans la formule (I), X représente un atome de soufre, on fait réagir une glycosylamine de formule (II) avec un S-ester de chlorure d'acide thiocarbonique $R^2—S—CO—Y$, dans lequel Y représente un atome d'halogène et $R^2$ a le sens indiqué à la revendication 1.

10. Procédé selon la revendication 1, caractérisé en ce que, si, dans la formule (I), X représente un groupe NH, on fait réagir une glycosylamine de formule (II) avec 1 à 5 équivalents d'un isocyanate $R^2—NCO$, dans lequel $R^2$ a le sens indiqué à la revendication 1, ou si, dans la formule (I), X représente un groupe NH ou un groupe N-alkyle, on fait réagir un carbamate (que l'on obtient par réaction de l'amine selon la formule (II) avec un ester aromatique d'acide halogénoformique) avec 1 à 10 équivalents d'une amine primaire $R^2—NH_2$ ou d'une amine secondaire $R^2$—NH-alkyle, dans laquelle $R^2$ a les sens indiqués à la revendication 1.